# EUROPEAN PATENT APPLICATION

(11) **EP 2 460 455 A1**
(43) Date of publication of application: **06.06.2012**
(21) Application number: 10804173.2
(22) Date of filing: 28.04.2010
(51) Int. Cl.: A61B 1/00

(54) **ACTUATOR SYSTEM AND ENDOSCOPIC DEVICE**

(30) Priority: 28.07.2009 JP 2009175637
(71) Applicant: OLYMPUS MEDICAL SYSTEMS CORP., Tokyo 151-0072 (JP)
(72) Inventor: YOSHIE, Michifumi, Tokyo 151-0072 (JP); KITAYAMA, Tadashi, Tokyo 151-0072 (JP)
(74) Representative: Schicker, Silvia
(86) International application number: PCT/JP2010/057567
(87) International publication number: WO 2011/013425

(57) **Abstract**

An actuator system 10 including a drive section 13 that performs driving by injection/suction of a liquid, a tube 12, a syringe pump 11 that injects/suctions the liquid into/from the drive section 13 via the tube 12, a pressure sensor 14 that measures an inner pressure, a storage section 56 that preliminarily stores a volume change ΔV of the tube 12 caused by the inner pressure, and a control section 57 that controls the syringe pump 11 based on the volume change ΔV, inner pressure P, and volume V0 of the liquid injected/suctioned into/from the drive section 13.

## Description

### Technical Field

The present invention relates to an actuator system provided with a drive section that performs driving by injection/suction of a fluid and an endoscope apparatus provided with such an actuator system, and more particularly, to an actuator system provided with a tube for supplying a fluid from a fluid supply section to a drive section and an endoscope apparatus provided with such an actuator system.

### Background Art

Fluid-driven actuator systems provided with a drive section that performs driving by injection/suction of a fluid can operate flexibly, and are therefore being increasingly used for robots, manipulators and medical devices or the like.

For example, Japanese Patent Application Laid-Open Publication No.2005-95989 proposes a robot arm using a McKibben type air actuator. Furthermore, Japanese Patent Application Laid-Open Publication No.2003-181780 discloses a manual manipulator that operates a liquid supply section from a manual operation section. Furthermore, Japanese Patent Application Laid-Open Publication No.2006-10904 discloses an endoscope apparatus provided with a bending portion that is bent by an air pressure actuator to measure a flow rate of a fluid injected/suctioned and an air pressure.

The fluid-driven actuator system is provided with basic components such as a drive section that performs driving by injection/suction of a fluid, a tube and a fluid supply section that injects/suctions the fluid into/from the drive section via the tube. The tube is a channel for the fluid and corresponds to an electric wire in an electric actuator, and therefore has a predetermined length so that the drive section and the fluid supply section are installed separately from each other.

The tube of the fluid-driven actuator system preferably has high rigidity so that the capacity, in other words, the volume of the fluid indwelling in the tube does not change under the pressure of the fluid therein. On the other hand, since the drive section is moved to a desired position away from the fluid supply section, the tube may be required to have flexibility, that is, easily bendable nature. However, the rigidity and the flexibility are contradictory properties and it is not easy for these properties to be compatible with each other. Further, the fluid pressure varies in accordance with magnitude of a load. That is, when a large load is exerted on the drive section, the fluid pressure is increased.

Therefore, in a fluid-driven actuator system provided with a flexible tube, it has not been easy to perform a control with high drive accuracy, i.e. high accuracy in a displacement amount or a force amount.

For example, the control apparatus disclosed in Japanese Patent Application Laid-Open Publication No.2005-95989 preliminarily stores a relationship between an amount of displacement and a fluid pressure in a memory as a table. The control apparatus controls the fluid pressure to a fluid pressure corresponding to a desired amount of displacement based on the table. The fluid supply section adjusts the fluid pressure to measure the amount of displacement and compensate for errors. However, as already explained, since the fluid pressure varies in accordance with the magnitude of the load, there has been a case where it is not easy to compensate the errors by the control performed by the fluid supply section based on the table.

Furthermore, the manual manipulator disclosed in Japanese Patent Application Laid-Open Publication No.2003-181780 uses a flexible pressure resistant tube. However, the flexibility and the pressure resistance are contradictory properties. Therefore, if the flexibility is regarded as important, the pressure resistance is deteriorated to increase a volume variation of the tube by the fluid pressure. On the other hand, if the pressure resistance is regarded as important, the thickness of the tube has to be set greater or a reinforcing member has to be increased, so that the diameter is increased and also the flexibility is deteriorated.

The endoscope apparatus disclosed in Japanese Patent Application Laid-Open Publication No.2006-10904 measures a flow rate of a fluid for the purpose of measuring temperature. The endoscope apparatus therefore uses a gas as a fluid whose volume changes with temperature.

An object of the present invention is to provide an actuator system capable of performing a control of high accuracy.

### Disclosure of Invention

### Means for Solving the Problem

In order to achieve the above object, an actuator system according to an embodiment of the present invention includes a drive section that performs driving by injection/suction of a fluid, a tube, a fluid supply section that injects/suctions the fluid into/from the drive section via the tube, a pressure measuring section that measures an inner pressure of the tube, a storage section that preliminarily stores a volume change of the tube caused by the inner pressure, and a control section that controls the fluid supply section based on the volume change stored in the storage section, the inner pressure measured by the pressure measuring section and a volume of the fluid injected/suctioned into/from the drive section by the fluid supply section.

In order to achieve the above object, an endoscope apparatus according to another embodiment of the present invention includes an actuator system comprising a drive section constituting a bending portion that performs driving by injection/suction of a fluid, a tube, a fluid supply section that injects/suctions the fluid into/from the drive section via the tube, a pressure measuring section that measures an inner pressure of the tube, a storage section that preliminarily stores a volume change of the tube caused by the inner pressure, and a control section that controls the fluid supply section based on the volume change stored in the storage section, the inner pressure measured by the pressure measuring section and a volume of the fluid injected/suctioned into/from the drive section by the fluid supply section.

### Brief Description of the Drawings

Fig. 1A is a diagram illustrating operation of an actuator system and illustrating a situation before drive;
Fig. 1B is a diagram illustrating operation of the actuator system and illustrating a case where the tube is not deformed;
Fig. 1C is a diagram illustrating operation of the actuator system and illustrating a case where the tube is inflated;
Fig. 1D is a diagram illustrating operation of the actuator system and illustrating a case where the tube is deflated;
Fig. 2 is a configuration diagram illustrating an overall configuration of an endoscope apparatus provided with the actuator system according to a first embodiment;
Fig. 3 is a perspective view of a multi-lumen tube making up a drive section of the actuator system according to the first embodiment;
Fig. 4A is a diagram illustrating the drive section of the actuator system according to the first embodiment;
Fig. 4B is a diagram illustrating the drive section of the actuator system according to the first embodiment;
Fig. 4C is a diagram illustrating the drive section of the actuator system according to the first embodiment;
Fig. 4D is a diagram illustrating the drive section of the actuator system according to the first embodiment;
Fig. 4E is a diagram illustrating the drive section of the actuator system according to the first embodiment;
Fig. 4F is a diagram illustrating the drive section of the actuator system according to the first embodiment;
Fig. 5 is a configuration diagram illustrating a configuration of the actuator system according to the first embodiment;
Fig. 6 is a diagram illustrating a relationship between the volume of the liquid outputted from a syringe pump and the volume of the liquid inputted to the drive section;
Fig. 7 is a diagram illustrating a relationship between a fluid pressure and the volume of the liquid inputted to the drive section;
Fig. 8 is a table illustrating a table of volume change of the tube caused by an inner pressure stored in the storage section; and
Fig. 9 is a diagram illustrating a catheter provided with an actuator system according to a second embodiment.

### Best Mode for Carrying Out the Invention

### <First embodiment>

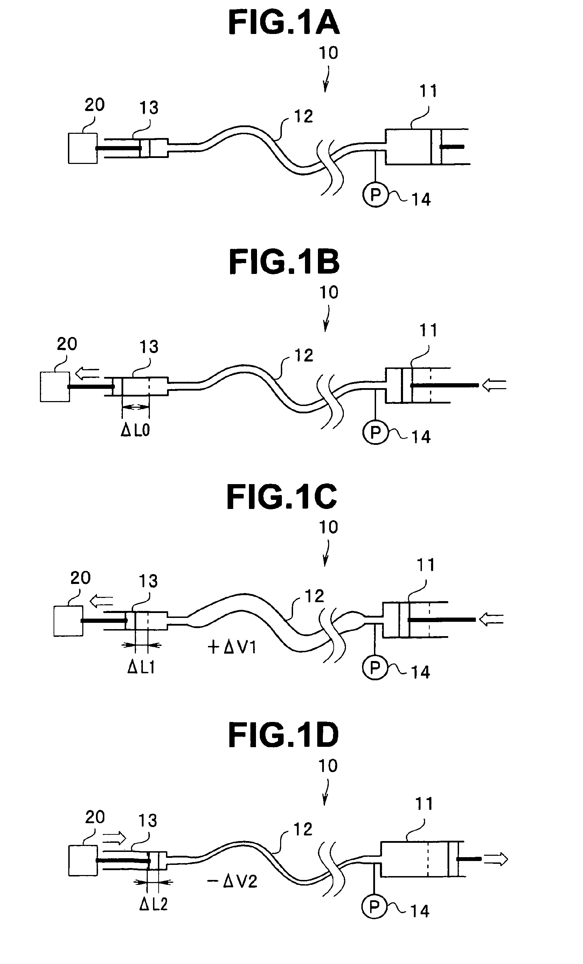

First, a relationship between deformation and accuracy of displacement of a tube 12 of an actuator system 10 according to a first embodiment will be described using Fig. 1A to Fig. 1D. As shown in Fig. 1A, the actuator system 10 of the present embodiment is provided with a drive section 13, a tube 12 which is a conduit, a syringe pump 11 which is a fluid supply section and a pressure sensor 14 which is a pressure measuring section. A liquid is injected/suctioned from the syringe pump 11 into/from the drive section 13 via the tube 12 and an external force is applied to a load 20. In Fig. 1A to Fig. 1D, the drive section 13 is described as a simple syringe type and deformation of the tube 12 is displayed exaggerated for ease of explanation.

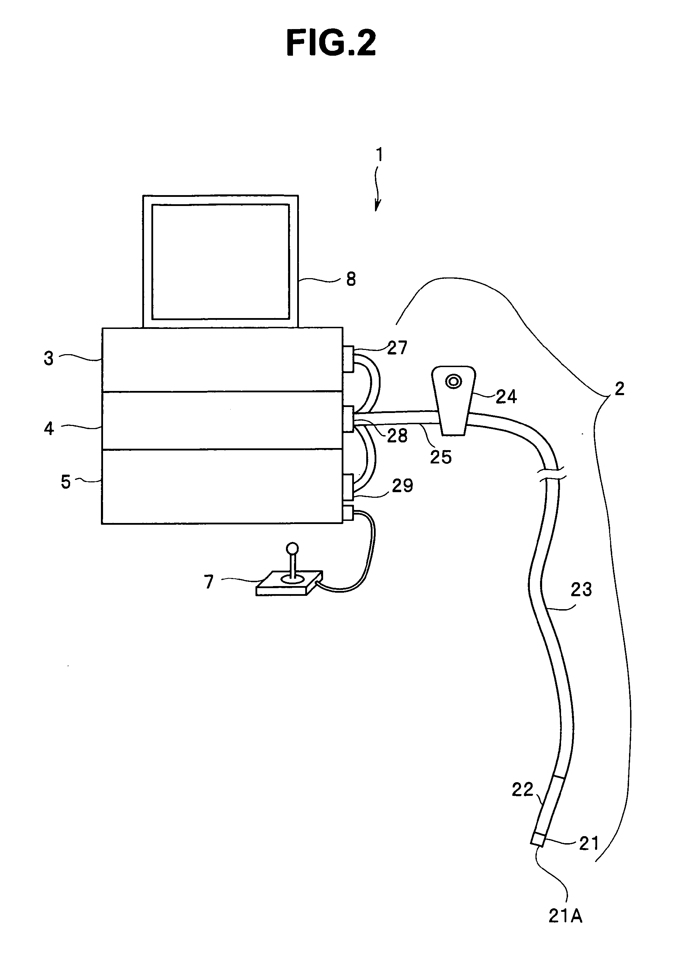

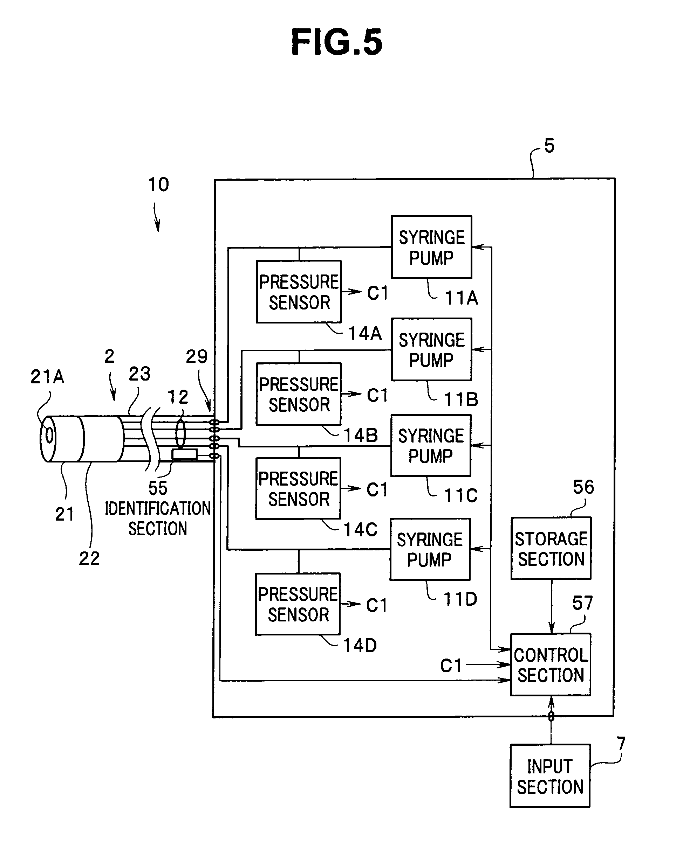

As shown in Fig. 1B, in the case where the tube 12 is not deformed by the liquid pressure, when the syringe pump 11 injects a liquid of predetermined volume V0 into the tube 12 under the control of a control section 57 (see Fig. 5) based on an input from an input section 7 (see Fig. 2), the drive section 13 is displaced by a predetermined amount of displacement ΔL0 corresponding to a volume V0 injected from the syringe pump 11. For example, assuming a cross-sectional area of the syringe of the drive section 13 is S, "ΔL0=V0/S." Thus, even if the control section controls the syringe pump 11 so as to output a volume of liquid to be supplied to the drive section 13, the drive accuracy does not deteriorate.

By contrast, the flexible tube 12 as shown in Fig. 1C is inflated and deformed when the internal liquid pressure increases under the influences of the load 20 or the like, and therefore the volume of the tube 12 increases by ΔV1. Therefore, the displacement amount ΔL1 of the drive section 13 becomes smaller than the displacement amount ΔL0 when the tube 12 is not deformed. That is, "ΔL1=(V0-ΔV1)/S." The displacement amount ΔL1 varies depending on the size of the load 20, that is, the liquid pressure. The ΔV1 varies depending on the material, thickness and length of the tube 12 and liquid pressure, but, for example, ΔV1=0.5×V0.

As shown in Fig. 1D, when the internal liquid pressure decreases under the influence of the load 20 or the like, the tube 12 is deflated and deformed, and therefore the volume of the tube 12 becomes smaller by ΔV2. Thus, the displacement amount ΔL2 of the drive section 13 becomes greater than the displacement amount ΔL0 when the tube 12 is not deformed. That is, "ΔL2=(V0+ΔV2)/S."

As described above, the actuator system that drives a fluid via a flexible tube has a problem that the drive accuracy degrades caused by deformation of the tube 12 under the liquid pressure. However, as will be described later, in the actuator system 10, the control section 57 (see Fig. 5) controls the syringe pump 11 in consideration of the deformation of the tube 12.

Next, the endoscope apparatus 1 provided with the actuator system 10 according to the first embodiment will be described using Fig. 2. As shown in Fig. 2, the endoscope apparatus 1 is provided with an endoscope 2, a CCU (camera control unit) 3, a light source apparatus 4, a bending control unit 5, the input section 7 and a monitor 8. As will be described later, the bending control unit 5 forms a part of the actuator system 10.

The endoscope 2 includes a distal end portion 21, a bending portion 22, an insertion portion 23, an operation section 24 and a universal cord 25 provided in that order from the distal end side of insertion. The distal end portion 21 of the endoscope 2 is provided with a CCD 21 A which is an image pickup device for picking up an image of an observation target inside a subject. A video signal captured by the CCD 21A is subjected to signal processing in the CCU 3 and an endoscope image is outputted to the monitor 8.

Furthermore, the distal end portion 21 is provided with an illumination optical system. That is, light generated by the light source apparatus 4 is guided to the distal end portion 21 via an LG (light guide) and is used as illumination light for observation using the CCD 21 A.

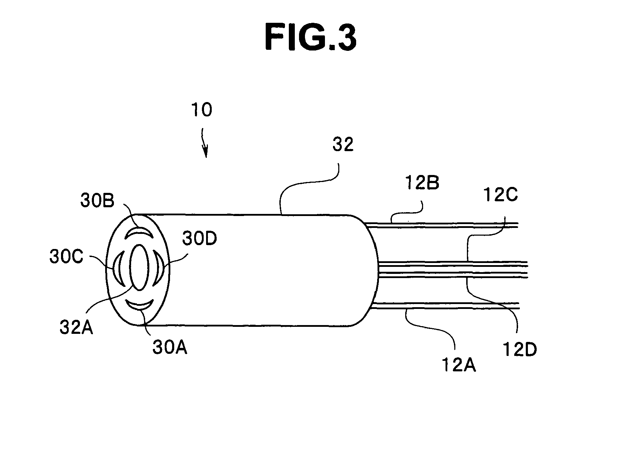

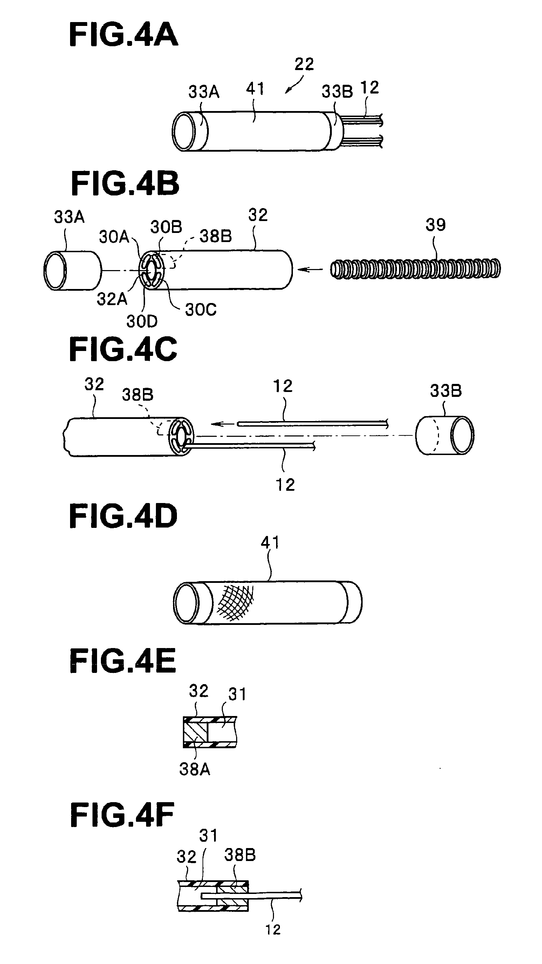

The bending control unit 5 injects/suctions a liquid into/from a multi-lumen tube 32 of the bending portion 22 inserted in the body of the subject (see Fig. 3, Fig. 4) via the tube 12 under the control of the control section 57 (see Fig. 5) according to the operation of the input section 7 by the operator.

The video signal cable, the LG and the tube 12 or the like pass through the universal cord 25 and are connected to the CCU 3, the light source apparatus 4 and the bending control unit 5 via a CCU connector 27, a light source apparatus connector 28 and a bending control unit connector 29 respectively.

Next, a configuration of the bending portion 22 will be described using Fig. 3 and Fig. 4A to Fig. 4F. The bending portion 22 has a multi-lumen tube 32 made up of four drive sections for bending in four directions put together and the four drive sections are connected with their respective tubes 12 for injecting/suctioning a liquid.

As shown in Fig. 3, the bending portion 22 has the multi-lumen tube 32 made of a flexible material such as silicone rubber and having a circular cross section and tubes 12A to 12D respectively connected to four peripheral lumens 30A to 30D having an arc-shaped cross section of the multi-lumen tube 32. Hereinafter, when a plurality of components having an identical function are referred to, the one alphabetic character at the end will be omitted. For example, when each of the four peripheral lumens 30A to 30D is indicated, it is referred to as "peripheral lumen 30" and when each of the four tubes 12A to 12D is indicated, it is referred to as "tube 12."

Next, the structure of the multi-lumen tube 32 will be further described using Fig. 4A to Fig. 4F. The drive section 13 is formed of the multi-lumen tube 32, an inner diameter regulating tube 39 which is an inner diameter regulating member and an outer diameter regulating blade 41 which is an outer diameter regulating member as main components. A center lumen 32A extends along an axial direction at the center position of a circular cross section of the multi-lumen tube 32. Internal items such as the aforementioned cable and LG are designed to be inserted through the center lumen 32A.

The four peripheral lumens 30 having an arc-shaped cross section are spaced substantially evenly in the circumferential direction on the tube wall around the center lumen 32A. Both front and rear ends of the four peripheral lumens 30 having the arc-shaped cross section are sealed with fillers 38A and 38B of silicone rubber as shown in Fig. 4E and Fig. 4F. That is, four hermetically sealed compartments 31A to 31D are formed using the four peripheral lumens 30.

A distal end portion of the tube 12 for injecting a liquid into each compartment 31 or suctioning a liquid from each compartment 31 is inserted in the filler 38B on the proximal end side of the endoscope of the four arc-shaped peripheral lumens 30.

Furthermore, a front base 33A and a rear base 33B are connected to the end of the distal end side and the end of the proximal end side of the multi-lumen tube 32 respectively by means of bonding or the like. The inner diameter regulating tube 39 as an inner diameter regulating member is inserted in the center lumen 32A of the multi-lumen tube 32.

The outside of the multi-lumen tube is covered with the outer diameter regulating blade 41 which is, for example, a cylindrically woven stainless steel wire as an outer diameter regulating member. Both ends of the outer diameter regulating blade 41 are fixed by solder at the positions corresponding to the front base 33A and the rear base 33B of the multi-lumen tube 32 respectively.

Furthermore, the outer circumferential face of the outer diameter regulating blade 41 is covered with a skin tube (not shown). The skin tube is made of a material such as fluoro rubber, urethane rubber, latex rubber. Both end portions of the skin tube are tied with a string at the positions of the front base 33A and the rear base 33B of the multi-lumen tube respectively and the outside of the string is fixed with an adhesive.

A bending operation of the bending portion 22 is performed by selectively injecting/suctioning the liquid into/from each compartment 31 of the four peripheral lumens 30 of the multi-lumen tube 32. In the present embodiment, each compartment 31 of the four peripheral lumens 30 is set so as to correspond to any one of the four bending directions, that is, leftward, rightward, upward and downward directions.

When the liquid is injected into any one of the compartments 31 of the peripheral lumen 30 of the multi-lumen tube 32 via the tube 12, the compartment 31 into which the liquid is injected increases in volume and is inflated outward and inward. However, the outer diameter regulating blade 41 as the outer diameter regulating member regulates the compartment 31 so as not to inflate outward. On the other hand, the inner diameter regulating tube 39 regulates the compartment 31 so as not to inflate inward. Thus, the compartment 31 can inflate neither outward nor inward, and thus extends in the axial direction. Therefore, the multi-lumen tube 32 is bent opposite to the compartment 31 into which the liquid is injected. To reduce the bending angle of the multi-lumen tube 32, the liquid inside may be suctioned.

Here, since the insertion portion 23 of the endoscope apparatus 1 is inserted along the tube cavity in the body of the subject, prime importance is placed on the flexibility thereof. Thus, it is important that the tube 12 inserted into the interior of the insertion portion 23 have flexibility, that is, have flexibility so as not to obstruct deformation of the insertion portion 23. Furthermore, the material of the tube 12 can be selected from among publicly known flexible materials in consideration of autoclave sterilization processing or the like and the tube 12 is made into a relatively thin hollow structure using a material such as polyolefin (e.g., polyethylene, polypropylene, ethylene-polypropylene copolymer), polyvinyl chloride, polyamide (e.g., nylon), polyimide, polyurethane, polyester, fluorine resin (e.g., polytetrafluoroethylene (PTFE), tetrafluoroethylene-hexafluoropropylene copolymer (FEP), silicone resin, silicone rubber, and preferably, silicone-based material.

Furthermore, water or oil or the like can be used as a drive liquid, but a physiological saline solution is preferably used. This is because if the liquid is released into the living body, damage to the subject will be small.

Next, the configuration of the actuator system 10 of the endoscope apparatus 1 will be described using Fig. 5. As has already been described, the actuator system 10 has four syringe pumps 11A to 11D and four pressure sensors 14A to 14D to drive the four drive sections independently of each other. In other words, the actuator system 10 is made up of four actuator systems sharing the bending control unit 5. Hereinafter, one syringe pump 11 and one pressure sensor 14 will be described for ease of explanation.

As has already been described, the actuator system 10 is an actuator to bend the bending portion 22 of the endoscope 2. The syringe pump 11 which is a fluid supply section injects a liquid into the compartment 31 of the insertion portion 23 via the tube 12. Here, the liquid only moves in a hermetically sealed space formed by the compartment 31, the tube 12 and the syringe pump 11 and will never be released to the outside of the system. Thus, unlike the actuator system that releases a drive fluid, the actuator system 10 need not continue to supply the fluid, and can thereby be downsized.

In the actuator system 10, the control section 57 controls the syringe pump 11 in consideration of deformation of the tube 12 due to a variation in the load 20. That is, the actuator system 10 is provided with a storage section 56 that preliminarily stores a volume change of the tube 12 caused by an inner pressure and the control section 57 controls the syringe pump 11 based on the volume change of the tube 12 stored in the storage section 56, the pressure measured by the pressure sensor 14 and the volume of the liquid injected/suctioned by the syringe pump 11 into/from the compartment 31 of the drive section.

As has already been described, the flexible tube 12 is inflated or deflated according to the inner pressure, that is, the liquid pressure, and the volume, that is, the volume of the indwelling liquid changes. When the volume of the tube 12 changes, even when the syringe pump 11 outputs, that is, pushes a predetermined volume of fluid, the volume of the fluid injected into the compartment 31 which is the drive section 13 changes.

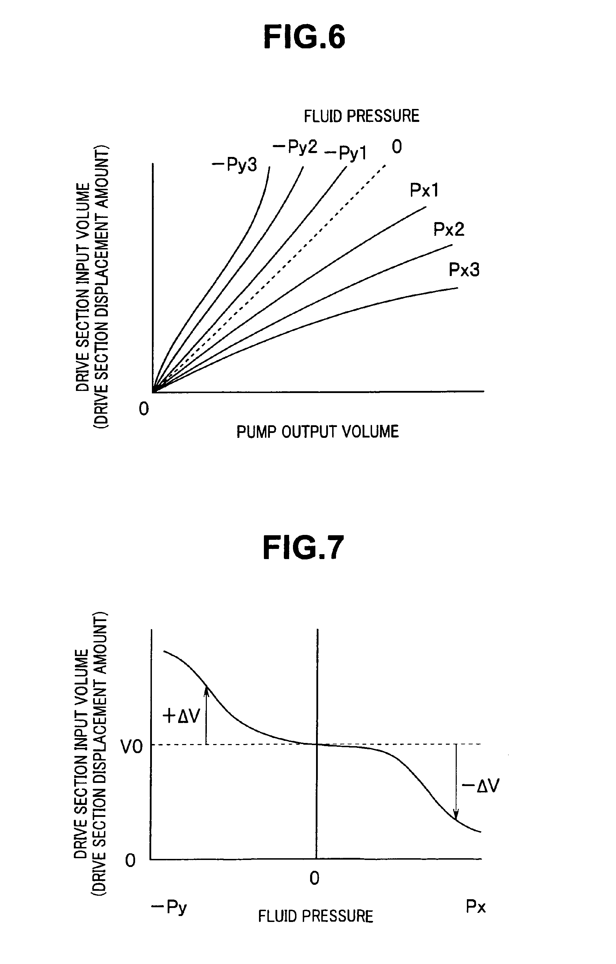

Fig. 6 illustrates a relationship between the volume of the liquid outputted from the syringe pump 11 and the volume of the liquid injected/suctioned into/from the drive section. As shown in Fig. 6, when the fluid pressure is 0, a pump output volume is equal to a drive section input volume. However, when a fluid is injected, as the fluid pressure increases to Px1, Px2, Px3, the drive section input volume with respect to the pump output volume decreases. On the contrary, in the case of suction, as the fluid pressure increases to -Py1, -Py2, -Px2 on the negative side, the drive section input volume with respect to the pump output volume increases. The volume of the liquid inputted (injected/suctioned) to the drive section 13 has a correlation with a displacement amount ΔL of the drive section.

Fig. 7 is a diagram illustrating the above described phenomenon from another viewpoint and illustrates the volume of the liquid actually inputted to the drive section 13 when the volume of the liquid outputted from the syringe pump 11 is a predetermined amount V0.

The volume change ΔV of the tube 12 caused by the inner pressure shown in Fig. 6 and Fig. 7 can be measured or calculated beforehand. Since rigidity of the tube 12 varies with temperature, the volume change caused by the inner pressure varies with temperature. Since the actuator system 10 is used in the body of the subject, it is preferable to use a volume change at a body temperature of the subject, for example, 36°C to 38°C.

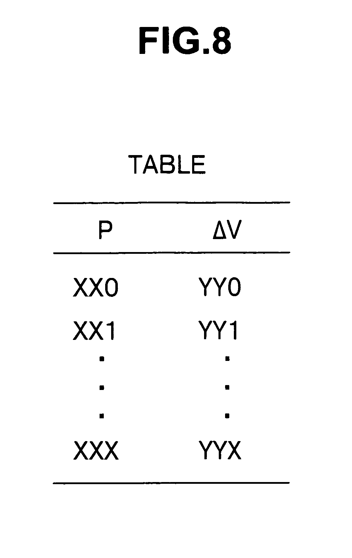

The volume change of the tube 12 caused by the inner pressure is stored in the storage section 56 as an equation using the inner pressure P as a parameter or as a table shown in Fig. 8.

In actuator system 10, the control section 57 controls the syringe pump 11 based on the information stored in the storage section 56 and the inner pressure P detected by the pressure sensor 14. Thus, even if the volume of the tube 12 varies due to a variation in load, the drive section 13 can perform control with high accuracy. Here, the accuracy refers to the accuracy of displacement amount of the drive section 13 or the accuracy of amount of drive force. The accuracy of displacement amount means that "ΔL1" and "ΔL2" described using Fig. 1(C) and Fig. 1(D) are small and the accuracy of drive force amount is the accuracy of the amount of force resulting from dividing the pressure P by the cross-sectional area of the drive section S.

As described above, the actuator system 10 of the endoscope apparatus according to the present embodiment can realize control with high accuracy even if the inner pressure of the tube 12 varies due to a variation in load or the like. Furthermore, since the flexible tube 12 is used, the actuator system 10 can use a tube of small diameter without impairing the ease of insertion of the insertion portion 23 and also contributes to a diameter reduction of the insertion portion.

As shown in Fig. 5 or the like, the endoscope 2, that is, the drive section 13 and the tube 12 are freely attachable or detachable to/from the bending control unit 5, that is, the syringe pump 11 which is a fluid supply section via a bending control unit connector 29 or the like. When a plurality of endoscope are provided and any one endoscope 2 is used, the endoscope 2, that is, the drive section 13 or tube 12 preferably has an identification section 55. The identification section 55 is a ROM that stores identification information with which the control section 57 can identify the type of the endoscope 2. The identification information stored in the identification section 55 may also be an electric resistance value of the identification section 55 itself or a barcode or the like that displays the type or the like as long as it can be identified by the control section 57.

In the endoscope apparatus 1 that can select an endoscope to be connected from among a plurality of endoscopes, the storage section 56 stores information on a volume change of the tube 12 corresponding to the type of each endoscope or the like, the control section 57 identifies the connected endoscope 2 based on the identification information of the identification section 55 and controls the syringe pump 11 based on the information on the volume change corresponding to the endoscope 2.

The actuator system 10 in the above described configuration can perform high accuracy control even when using any one of a plurality of endoscopes, and so provides a high level of convenience.

### <Second embodiment>

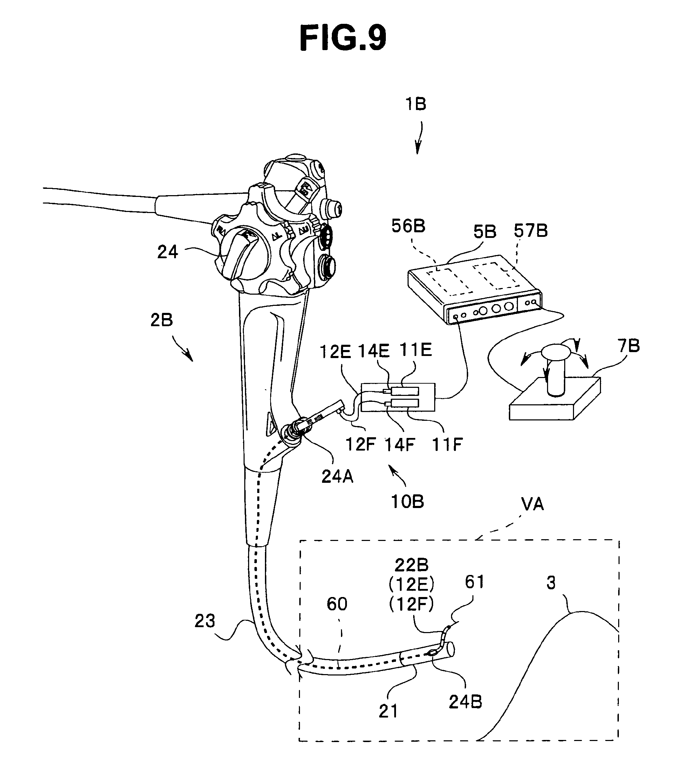

Next, an active catheter (hereinafter referred to as "catheter") 60 provided with an actuator system 10B of a second embodiment will be described using Fig. 9. As shown in Fig. 9, the catheter 60 is inserted from a treatment instrument insertion hole 24A disposed in the vicinity of an operation section 24 of an endoscope 2B into a treatment instrument channel disposed inside an insertion portion 23B and goes out of a treatment instrument outlet 24B of the distal end portion. The catheter 60 then performs treatment of a tissue 3 in the body (VA) of the subject, for example, extraction of the tissue. The catheter 60 has a bending portion 22B for bending a distal end portion 61 through operation of an input section 7B. The catheter 60 is used by inserting a distal end portion 21 of the endoscope 2B up to the vicinity of the tissue 3 in the body of the subject VA and then making it go out of the treatment instrument outlet 24B.

Since the bending portion 22B can be bent in two directions, the actuator system 10B has two syringe pumps 11E and 11F, two pressure sensors 14E and 14F, two tubes (12E, 12F), two drive sections (13E, 13F), a storage section 56B and a control section 57B, but only one drive section or the like will be described below for ease of explanation. The basic configuration of the drive section 13 is the same as that in Fig. 1 or Fig. 3, but the actuator system 10B uses a gas, for example, air as a drive fluid.

That is, in the actuator system 10B, air is sealed in a hermetically sealed space formed by the syringe pump 11, the flexible tube 12 and the drive section 13. Here, unlike a liquid, air is an elastic fluid and varies in volume depending on a pressure. That is, when a uniform pressure P is applied to air, which is the elastic fluid, the volume thereof decreases at a rate of P/k. This k is referred to as a "bulk modulus," which is a matter-specific constant.

The storage section 56B in the actuator system 10B preliminarily stores not only a volume change of the tube 12 caused by an inner pressure but also a bulk modulus of air. The control section 57B then controls the syringe pump 11 based on the volume change of the tube 12 and bulk modulus of air stored in the storage section 56B, the inner pressure measured by the pressure sensor 14 and the volume of air injected/suctioned into/from the drive section 13 by the syringe pump 11A.

Thus, in addition to the effects of the actuator system 10 of the first embodiment, although the actuator system 10B uses air which is the elastic fluid as the drive fluid, the drive accuracy never deteriorates due to a volume variation of air caused by a variation in pressure.

The present invention is not limited to the aforementioned embodiments. For example, the embodiments have described the actuator system provided with a control section that controls a plurality of syringe pumps so as to inject/suction a fluid into/from the respective drive sections via the respective tubes, but it goes without saying that an actuator system may also be provided with a control section that controls one syringe pump so as to inject/suction a fluid into/from one drive section via one tube.

Furthermore, since an actuator system of an active catheter used as a single unit is required to have a small diameter and high flexibility so as to be inserted into body cavities (blood vessel, ureter, oviduct, bile duct, pancreatic duct or the like) having a fine and complicated pattern percutaneously, nasotracheally or orally speedily and with reliable selectivity, the actuator system of the present invention can be preferably used.

Furthermore, a liquid may also be used as the drive fluid in the actuator system of the active catheter as described in the first embodiment. A physiological saline solution can be preferably used for the active catheter used particularly in a blood vessel.

Furthermore, the drive section needs only to be made drivable by a drive fluid, and the drive section can be a mechanism that transmits the movement of the syringe-type movable section to a load via a wire as shown in Fig. 1. Furthermore, the storage section and the control section may be part of, for example, a CPU that controls the entire endoscope apparatus.

Furthermore, a case has been described where the actuator system is used for the endoscope 2 and the active catheter 60, that is, used for medical devices, but the present invention is not limited to this.

That is, the present invention can be changed, modified or the like in various ways without departing from the spirit and scope of the present invention.

As described so far, the endoscope apparatus according to the embodiments of the present invention is provided with an actuator system including a drive section making up a bending portion that performs driving through injection/suction of a liquid, a flexible tube, a syringe pump that injects/suctions the liquid into/from the drive section via the tube, a pressure measuring section that measures an inner pressure of the tube, a storage section that preliminarily stores a volume change of the tube caused by the inner pressure, and a control section that controls the syringe pump based on the volume change stored in the storage section, the inner pressure measured by the pressure measuring section and the volume of the liquid injected/suctioned into/from the drive section.

The present application is filed claiming the priority of Japanese Patent Application No. 2009-175637 filed in Japan on July 28, 2009, and the above described disclosure is incorporated by reference in the present description, claims and drawings.

## Claims

1. An actuator system comprising:
a drive section that performs driving by injection/suction of a fluid;
a tube;
a fluid supply section that injects/suctions the fluid into/from the drive section via the tube;
a pressure measuring section that measures an inner pressure of the tube;
a storage section that preliminarily stores a volume change of the tube caused by the inner pressure; and
a control section that controls the fluid supply section based on the volume change stored in the storage section, the inner pressure measured by the pressure measuring section and a volume of the fluid injected/suctioned into/from the drive section.

2. The actuator system according to claim 1, wherein the fluid supply section is a syringe pump that can measure a volume of the fluid injected/suctioned into/from the tube.

3. The actuator system according to claim 1, wherein the fluid moves in a hermetically sealed space formed by the drive section, the tube and the fluid supply section.

4. The actuator system according to claim 1, wherein the fluid supply section is disposed outside a body of a subject and injects/suctions the fluid into/from the drive section inserted in the body of the subject via the tube.

5. The actuator system according to claim 4, wherein the volume change stored in the storage section is a temperature value in the body of the subject.

6. The actuator system according to claim 1, wherein the tube is freely attachable/detachable to/from the fluid supply section,
the drive section or the tube comprises an identification section that stores identification information, and
the control section controls the fluid supply section based on the volume change, the inner pressure, the volume of the fluid injected/suctioned into/from the drive section and the identification information.

7. The actuator system according to claim 1, wherein the fluid is a liquid.

8. The actuator system according to claim 7, wherein the liquid is a physiological saline solution.

9. The actuator system according to claim 1, wherein the fluid is a gas,
the storage section preliminarily stores a bulk modulus of the gas, and
the control section controls the fluid supply section based on the volume change and the bulk modulus stored in the storage section, the inner pressure measured by the pressure measuring section and the volume of the fluid injected/suctioned into/from the drive section.

10. An endoscope apparatus including an actuator system comprising:
a drive section constituting a bending portion that performs driving by injection/suction of a fluid;
a tube;
a fluid supply section that injects/suctions the fluid into/from the drive section via the tube;
a pressure measuring section that measures an inner pressure of the tube;
a storage section that preliminarily stores a volume change of the tube caused by the inner pressure; and
a control section that controls the fluid supply section based on the volume change stored in the storage section, the inner pressure measured by the pressure measuring section and a volume of the fluid injected/suctioned into/from the drive section.

11. The endoscope apparatus according to claim 10, wherein the fluid supply section is a syringe pump that can measure the volume of the fluid injected/suctioned into/from the tube.

12. The endoscope apparatus according to claim 10, wherein the fluid moves in a hermetically sealed space formed by the drive section, the tube and the fluid supply section.

13. The endoscope apparatus according to claim 10, wherein the fluid supply section is disposed outside a body of a subject and injects/suctions the fluid into/from the drive section inserted in the body of the subject via the tube.

14. The endoscope apparatus according to claim 10, wherein the liquid is a physiological saline solution.
